# EUROPEAN PATENT SPECIFICATION

(11) **EP 2 031 986 B1**
(45) Date of publication and mention of the grant of the patent: **20.03.2013**
(21) Application number: 07786738.0
(22) Date of filing: 14.06.2007
(51) Int. Cl.: A23L 1/305, A61K 38/01, A61P 37/08

(54) **INDUCTION OF TOLERANCE TO EGG PROTEINS**
INDUKTION DER VERTRÄGLICHKEIT ZUR EIPROTEINEN
INDUCTION DE LA TOLÉRANCE AUX PROTÉINES D'OEUF

(30) Priority: 15.06.2006 EP 06115533
(43) Date of publication of application: 11.03.2009
(73) Proprietor: Nestec S.A., 1800 Vevey (CH)
(72) Inventor: FRITSCHE, Rodolphe, 1814 La Tour-de-peilz (CH); SCHALLER, Raphael, 1066 Epalinges (CH)
(74) Representative: Cogniat, Eric Jean Marie
(86) International application number: PCT/EP2007/055882
(87) International publication number: WO 2007/144397

(56) References cited:
- EP-A- 0 629 350
- EP-A1- 0 827 697
- WO-A-97/00078
- US-A1- 2002 037 357
- FRITSCHE ET AL: "Induction of systemic immunologic tolerance to beta-lactoglobulin by oral administration of a whey protein hydrolysate" JOURNAL OF ALLERGY AND CLINICAL IMMUNOLOGY, MOSBY - YEARLY BOOK, INC, US, vol. 100, no. 2, August 1997 (1997-08), pages 266-273, XP005189327 ISSN: 0091-6749
- DATABASE BIOSIS BIOSCIENCES INFORMATION SERVICE, PHILADELPHIA, PA, US; Brazil. J. Med. Biol. Res. 1998, 31 (3): 381-386 1998, STRANSKY B, FARIA AMC, VAZ NM: "Oral tolerance with altered forms of ovalbumin" XP002402551 Database accession no. PREV199800276511

## Description

### Field of the Invention

This invention relates to the use of hydrolysed egg proteins to induce oral tolerance to intact egg proteins in mammals likely to be allergic to eggs.

### Background to the Invention

Food allergies, of which the most common is cows' milk allergy, are caused, in most cases, by a reaction to the proteins in the food. In the early years of life the immune system is still developing and may fail to recognise and tolerate such dietary proteins. The result is that the baby or child or young animal treats the dietary protein as a foreign substance and develops an allergic response to it. Food allergies may affect not only humans but also other mammals such as dogs and cats.

T helper cells play a central role in adaptive immunity. Th1 cells are vital for cell-mediated immune responses, and Th2 cells promote humoral immunity. Th1 and Th2 responses are counter-regulative, that is, cytokines produced by Th1 cells inhibit Th2 function and vice versa. Th2-skewed immune response has been shown to be crucial for the maintenance of successful pregnancy and it also prevails at birth and during the first months of life. Postnatal exposure to microbial antigens elicits preferentially Th1 responses, which have been suggested to counterbalance Th2-polarized cytokine production in neonates. In the case of insufficient early microbial exposure, the production of Th2-type cytokines (IL-4, IL-5 and IL-13) is further propagated leading to IgE production and consequently to allergic disease. However, this Th2 paradigm rapidly proved to be insufficient to explain the whole immunopathology of atopic disease and recently it was hypothesized that rather than an increased Th2 activation, the initial stages of atopic diseases could be a consequence of defective activation of T regulatory (Treg) cells. Treg cells are small T cell populations able to induce immune tolerance. Several overlapping subsets of Treg cells have been described (Th3, Tr1, CD4+,CD25+) expressing suppressive cytokines (IL-10, TGF-β).

The phenomenon of oral tolerance is the ability by which administration of antigens by the oral route can prevent subsequent systemic immune responses to the same antigen given in an immunogenic form. If the mechanism of oral tolerance does not develop sufficiently, or if there is a breakdown in the physiological state of tolerance to certain antigens, this may result in the development of hypersensitivity reactions. The mechanism can be explained as follows: following a first contact with the allergen, IgE antibodies are produced and migrate to the surface of mast cells and basophils where they are bound to specific receptors. Upon a second contact with the allergen, surface IgE are cross-linked on mast cells or basophils leading to cell activation and release of chemical mediators, including histamine. This phenomenon leads to pathologic effects, such as local or systemic vasodilatation.

Usually, food hypersensitivity appears just after a susceptible baby, child or young animal first encounters a new food. The first dietary proteins generally encountered by human babies at least are cows' milk proteins and, as noted above, cows' milk allergy is the most common food allergy. It is generally accepted that babies with established cows' milk allergy have an increased risk of developing allergies to other dietary proteins such as egg and cereal proteins but even those babies who have successfully developed oral tolerance to cows' milk proteins may subsequently develop allergies to other dietary proteins such as egg and cereal proteins when these are introduced into the diet at weaning.

From a dietary point of view there are two ways to treat an established allergy - either foods containing the allergen must be avoided altogether, or the foods must be treated to decrease their allergenic potential, for example by extensive hydrolysis. Infant formulas containing extensively hydrolysed cows' milk proteins (peptides consisting of not more than five amino acids) are manufactured for this latter purpose.

However, there is a need for products that help to reduce the risk of developing the allergy and promote the development of tolerance to intact proteins, particularly in children thought to be at risk of the same (that is, children having at least one close family member who suffers from an allergy). For example, it has been proposed to feed partially hydrolysed cows' milk proteins to induce oral tolerance to cows' milk proteins in infants. Fritsché et al. (J. Allergy Clin. Immunol, Vol 100, No.2, pages 266-273) have shown using animal models that enzymatic hydrolysates of cow's milk proteins with a degree of hydrolysis of 18% were able to induce oral tolerance to intact cow's milk proteins whereas hydrolysates with a degree of hydrolysis of 28% were not. Results of these experiments showed that preventive feeding of rats with such a moderately hydrolysed cow's milk formula, whose allergenicity had been reduced over 100 times as compared to a standard formula, suppressed specific IgE and mediator release from intestinal mast cells, both parameters of an immediate type allergic reaction. This work demonstrated that for cows' milk proteins it is possible to define a degree of enzymatic hydrolysis whereby the capacity of the peptides to induce oral tolerance is maintained whilst their allergenicity is substantially reduced.

Various other approaches have been proposed to improve induction of oral tolerance to cows' milk proteins including administration of probiotics as proposed in WO2003/099037 or administration of a compound capable of increasing COX-2 activity as proposed in WO02/051437. However, relatively little attention has been paid to induction of tolerance to other dietary proteins which frequently provoke allergic reactions such as egg proteins. Indeed, this may be an even greater need given that allergy to cows' milk proteins usually disappears spontaneously between the age of two and five years whereas allergy to egg proteins is generally slower to disappear and may even persist throughout life. It is therefore an object of the present invention to provide a method of inducing oral tolerance to egg proteins

### Summary of the Invention

Accordingly, the present invention provides the use of enzymatically hydrolysed egg proteins with a degree of hydrolysis between 15 and 28% in the manufacture of a composition for induction of oral tolerance to egg proteins in a mammal.

The invention extends to a method of inducing oral tolerance to egg proteins by providing to a mammal in need thereof a composition containing a therapeutic amount of hydrolysed egg proteins with a degree of hydrolysis between 15 and 28%.

### Brief Description of the Drawings

Figure 1 shows the residual OVA-specific antigenicity of egg hydrolysates
Figure 2 shows the reduced allergenicity in a functional mast cell triggering assay
Figure 3 shows the ability of different egg protein hydrolysates to suppress a specific IgE anti-egg protein response
Figure 4 shows the ability of different egg hydrolysates to down-regulate triggering of intestinal mast cells
Figure 5 shows that extensively hydrolysed egg proteins are unable to suppress a specific IgE anti-egg protein response or to down-regulate triggering of intestinal mast cells

### Detailed Description of the Invention

In this specification, the following terms have the following meanings:-
"degree of hydrolysis" or "DH" of a protein means the amount of nitrogen in free NH₂ groups divided by the total amount of nitrogen (NH and NH₂ groups) expressed as a percentage
"oral tolerance " means an active state of immunological hyporesponsiveness to antigens delivered via the oral route.

All references to percentages are percentages by weight unless otherwise stated.

Preferably the degree of hydrolysis is between 18 and 25%, more preferably between 23 and 25%.

The successful induction of oral tolerance to intact egg proteins using hydrolysed egg proteins requires a balance to be struck between the residual antigenicity of the hydrolysed proteins and their capacity to induce oral tolerance. In general, the residual antigenicity of the hydrolysed proteins should be at least 100 times less than that of the intact proteins.

It is found that hydrolysed egg proteins having a degree of hydrolysis between 20 and 28% have an allergenicity which is reduced by a factor of at least 100 compared to intact egg proteins as measured by the technique described by Fritsché et al (Int. Arch. Aller and Appl Imm., 93, 289-293, 1990).

The egg proteins may be enzymatically hydrolysed by any suitable process known in the art. One example of a suitable hydrolysis process is a two stage enzymatic hydrolysis starting from pasteurised liquid whole egg. The liquid egg is heated to a temperature in the range from 60 to 65°C for about 10 minutes, then cooled to about 55°C. A protease such as the bacterial serine endoprotease subtilisin (sold for example under the trade mark Alcalase®) is added and the mixture is maintained at about 55°C for at least two hours to effect a partial hydrolysis. Then the temperature of the mixture is raised to 70 to 75°C and held there for about 10 minutes. The mixture is again cooled to about 55°C and a further amount of enzyme is added. The mixture is maintained at about 55°C for at least a further two hours to achieve the required degree of hydrolysis. The temperature is then raised to between 85 and 95°C and held there for a period of up to 30 minutes to inactivate the enzymes and terminate the hydrolysis. The resulting liquid hydrolysed egg may be used in this state or may be spray dried to produce a powdered product as preferred.

A composition suitable for use in the present invention may be any food product in which whole egg is conventionally incorporated with the whole egg replaced by hydrolysed egg in which the egg proteins have a degree of hydrolysis between 20 and 28%. Hydrolysed egg powder produced as described above for example may be used in place of whole egg powder in recipes such as baked custards, quiches, crème caramel. Alternatively, the hydrolysed egg powder may be reconstituted with water and used to prepare dishes such as omelettes and scrambled eggs. Hydrolysed egg powder is a particularly suitable ingredient in foods for babies and small children, particularly foods suitable for use in the early stages of weaning. Again, the hydrolysed egg powder may be used in place of the whole egg powder conventionally used to prepare such products.

As noted above, allergies to dietary proteins are not confined to humans and the method of the present invention may also be used to induce oral tolerance to egg proteins in other mammals, particularly companion animals such as dogs and cats. Hydrolysed egg proteins with a degree of hydrolysis between 15 and 28% may thus also be used to replace whole egg in foods for companion animals, particularly foods intended for weaning puppies and kittens for example.

The invention will now be further described with reference to the following examples.

### Preparation of Egg Hydrolysates

The starting material was pasteurised liquid whole egg, FT/OVO/0105 R, ABCD S.A., Avicole Bretonne Cecab Distribution (Ploërmel, France).

### Example 1

30Kg of liquid whole egg was heated at 65°C for 10 min with stirring at 250 rpm. After cooling to 55°C, 2% of Protamex® enzymes (batch PW2A1006, NOVOZYMES A/S Bagsvaerd, Denmark) was added and the mixture was maintained at 55°C for 2 hours. After this first hydrolysis step, 1% of Flavourzyme® 1000 L enzymes (batch 400904, NOVOZYMES A/S Bagsvaerd, Denmark) was added and the mixture was heated at 75°C for 10 min. The mixture was then cooled to 55°C, a further 1% of Flavourzyme enzymes was added and the mixture was maintained at 55°C for 2 hours. After this second hydrolysis step, the mixture was heated at 90°C for 30 min and then spray-dried to obtain a hydrolysed egg powder which was conditioned in an aluminium bag.

### Example 2

35Kg of liquid whole egg was heated at 65°C for 10 min with stirring at 250 rpm. After cooling to 55°C, 5% of Protamex® enzymes (batch PW2A1006, NOVOZYMES A/S Bagsvaerd, Denmark) was added and the mixture was maintained at 55°C for 2 hours. After this first hydrolysis step, 1% of Flavourzyme® 1000 L enzymes (batch 400904, NOVOZYMES A/S Bagsvaerd, Denmark) was added and the mixture was heated at 75°C for 10 min. The mixture was then cooled to 55°C, a further 4% of Flavourzyme enzymes was added and the mixture was maintained at 55°C for 2 hours. After this second hydrolysis step, the mixture was heated at 90°C for 30 min and then spray-dried to obtain a hydrolysed egg powder which was conditioned in an aluminium bag.

### Example 3

30Kg of liquid whole egg was heated at 65°C for 10 min with stirring at 250 rpm. After cooling to 55°C, 10% of Alcalase®2.4L enzymes (batch 500357, NOVOZYMES A/S Bagsvaerd, Denmark) was added and the mixture was maintained at 55°C for 2 hours. After this first hydrolysis step, the mixture was heated at 75°C for 10 min. The mixture was then cooled to 55°C, a further 10% of Alcalase enzymes was added and the mixture was maintained at 55°C for 2 hours. After this second hydrolysis step, the mixture was heated at 90°C for 30 min and then spray-dried to obtain a hydrolysed egg powder which was conditioned in an aluminium bag.

### Products Containing Hydrolysed Whole Egg

### Example 4

An example of the ingredients for a sweet egg pudding containing hydrolysed egg is as follows:-

| **Ingredient** | **%** |
|---|---|
| Whole milk (3.5% fat) | 62.0 |
| Water | 24.3 |
| Sugar | 5.5 |
| Hypoallergenic egg powder | 2.5 |
| Corn starch | 3.0 |
| Tapioca starch | 2.0 |
| Vanilla flavouring | 0.7 |

The pudding may be made by any suitable method known in the art.

### Example 5

An example of the ingredients for a savoury egg pudding containing hydrolysed egg is as follows:-

| **Ingredient** | **%** |
|---|---|
| Whole milk (3.5% fat) | 62.0 |
| Water | 21.5 |
| Frozen carrot cubes | 10.0 |
| Hypoallergenic egg powder | 1.5 |
| Corn starch | 3.0 |
| Tapioca starch | 2.0 |

The pudding may be made by any suitable method known in the art.

### Example 6

An example of the ingredients for an egg pasta product containing hydrolysed egg is as follows:-

| **Ingredient** | **%** |
|---|---|
| Durum wheat semolina | 70.6 |
| Water | 21.6 |
| Hypoallergenic egg powder | 5.9 |
| Sunflower oil | 1.9 |

The pasta may be made by any suitable method known in the art.

### Residual Antigenicity of Egg Hydrolysates

The residual antigenicity of the protein ovalbumin (OVA) in the hydrolysates of Examples 1, 2 and 3 was determined by ELISA inhibition with a polyclonal rabbit anti-OVA protein antiserum. Wells of microtitration plates were coated with 100µl of OVA at 50 µg/ml in carbonate-bicarbonate buffer and incubated 24 hours at 4°C. Plates were washed 4 times in a PBS-Tween buffer and free reacting sites were blocked by adding 200µl/well of fish gelatin (0.5 % in PBS-Tween). Plates were incubated I hour at room temperature (RT) and washed again 4 times in PBS-Tween.

In separate tubes, 1 part of a standard OVA preparation or test sample are incubated for 1 hour at RT with 1 part of rabbit anti-OVA protein antibody (diluted 1:20'000). After incubation, 100 µl of this inhibition mixture is added to the above coated and blocked microtitration wells and incubated for 2 hours at room temperature. Plates were washed 4 times in PBS-Tween. A goat anti-rabbit peroxidase labelled conjugate (0.1 ml of a 1:2000 dilution) was then added, plates were incubated for 1 hour at room temperature and washed 4 times in PBS-Tween. The chromogenic substrate (0.1 ml 0-phenylene-diamine) was added. After 15 minutes incubation, optical density was read at 492 nm on an ELISA plate reader.

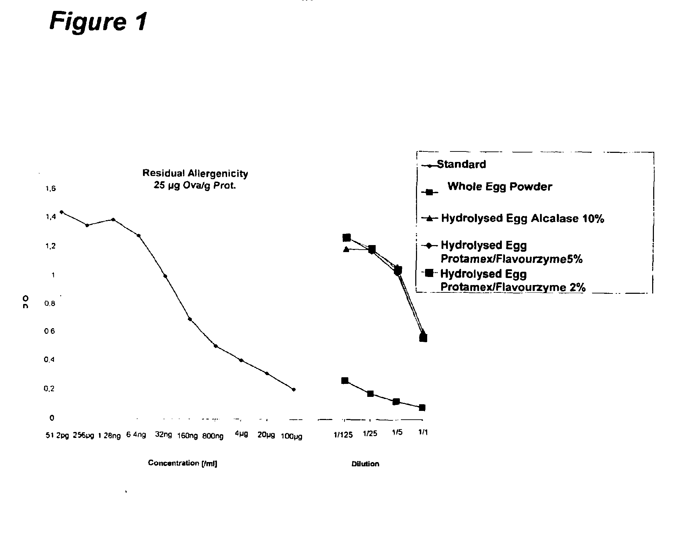

The results are shown in Figure 1 from which it may be seen that the OVA specific antigenicity of the hydrolysates from Examples 1 to 3 was reduced by a factor of over 10,000 compared to intact egg protein.

### Residual Allergenicity of Egg Hydrolysates

A functional in vitro assay of tritiated serotonin release from sensitised rat mast cells was used to determine IgE dependent allergenicity of an antigenic molecule (OVA) as previously described (Fritsché et al. J. Allergy Clin. Immunol, Vol 100, No.2, pages 266-273). Briefly, mast cells were obtained from normal Sprague-Dawley rats by peritoneal washes in Dulbecco's modified Eagle's medium containing 10% fetal calf serum. Cells were washed in this medium and kept overnight at 4°C. After two washes in phosphate-HEPES-fish gelatine buffer (PHG) pH 7.0, cells were re-suspended in the same buffer at 5x10⁵ cells/ml and diluted with one volume of rat serum rich in IgE anti-OVA antibodies containing 5 µCi/ml ³H serotonin. After incubation at 37°C for 2 hours, cells were further washed three times in PHG and re-suspended in PHG at 2.5x10⁵ cells/ml. Sensitised mast cells were distributed in microtiter plates (0.1 ml/well) and mixed to 0.05 ml of serial dilutions of hydrolysed egg proteins produced according to Example 2 (1/10 starting at 10 mg/ml). The mixture was incubated 60 minutes at 37°C and centrifuged. An aliquot (0.05ml) of the supernatant was mixed with 2 ml of scintillation fluid and ³H release was measured using a Packard β-counter.

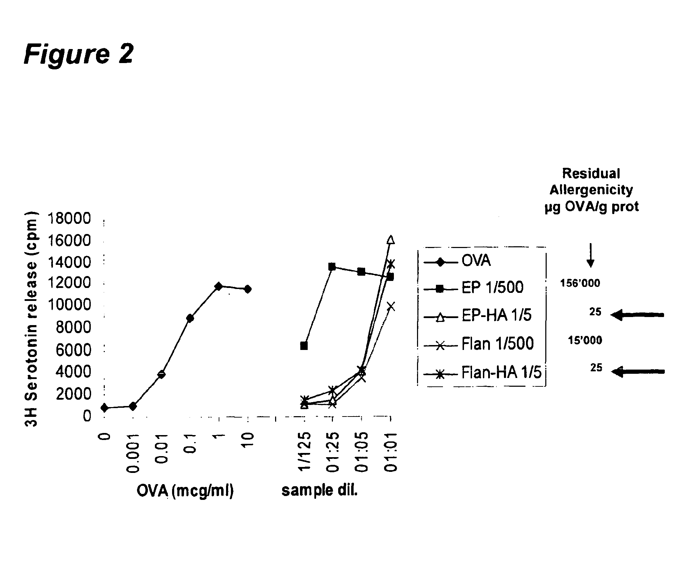

The results are shown in Figure 2 from which it may be seen that the hydrolysed egg had a much reduced allergenicity (25 µg OVA/g protein equivalent) and that this low value was maintained when the hydrolysed egg was incorporated in a flan style dessert.

### Induction of Oral Tolerance to Egg Proteins by Feeding Egg Protein Hydrolysate

The oral tolerance inducing capacity of egg products was investigated using an *in vivo* rat model. Six groups of Sprague-Dawley rats (6 animals/group) raised on an egg protein free diet were given different experimental liquid egg proteins /egg hydrolysates or water (control) *ad libitum* in their drinking bottles and a solid egg protein free pellet diet from days 1 to 19 of the experiment. Animals were given the following products:
Group A, whole egg powder (20g/l);
Group B, hydrolysed egg powder from Example 3 (120 g/l), DH 25%;
Group C, hydrolysed egg powder from Example 2 (120 g/l DH 23%;
Group D, hydrolysed egg powder from Example 1 (120 g/l) DH 20%;
Group E, ultrafiltrated hydrolysed egg powder from Example 3 (120g/l) DH 31 %;
Group F, H₂O (control).

The ultrafiltrated hydrolysed egg powder fed to Group E was obtained as follows. The substrate hydrolysed liquid egg obtained in Example 3 was microfiltrated using a filtration module (Sefiltec, FBF 0102) with a bag filter 100µm (PGF 51 E 02). After this microfiltration step, the permeate was ultrafiltrated using a home made UF module with 4000 Daltons membranes (ES404, PES, 4000 MWCO, PCI Membrane Systems). The permeate was then freeze-dried.

Total nitrogen in the hydrolysates was determined by the Dumas procedure (Carlo Erba method). Degree of hydrolysis was measured by the TNBS method according to Adler-Nissen (J. Agric. Food. Chem. 1979 27: 1256-1262).

All rats were immunized on day 5 of the experiment by subcutaneous injection of 0.1 mg Ovalbumin + 0.2 ml 3% Al(OH)3. On day 19, all animals were killed. Blood was drawn and sera were analysed for specific IgE antibodies (anti-Ovalbumin) by ELISA, as previously described (Fritsché R, Bonzon M. Int Arch Allergy Appl Immunol 1990;93:289-93). In brief, microtiter plates were coated with OVA for 24 hours at 4°C. Plates were washed with PBS Tween 20 and saturated for 1 h with fish gelatine. After addition of test sera serially diluted (1/2) and incubation for 2 h, a sheep anti- rat IgE antiserum was added. After 1 hour at room temperature, a peroxidase-labelled second antibody was added for 1 hour, followed by the substrate (o-phenylene-diamine). The optical density of a 1:5 diluted pool of normal rat sera at 492 nm was considered as non-specific background. Concentrations of specific antibodies were expressed as maximum dilutions of test sera above this value.

Rat mast cell protease (RMCPII) is released into blood following IgE mediated triggering of intestinal mast cells. Oral challenge for release of RMCPII is a measure of IgE sensitization or tolerization at the intestinal mast cell level.

RMCPII levels are determined with a commercial ELISA kit (Moredun Animal Health Ltd., Edinburgh, Scotland) based on the sandwich test principle in which the plate coating is made with a monoclonal anti-RMCPII antibody, followed by the addition of test serum and a second sheep anti-RMCPII polyclonal antibody coupled to horseradish peroxidase.

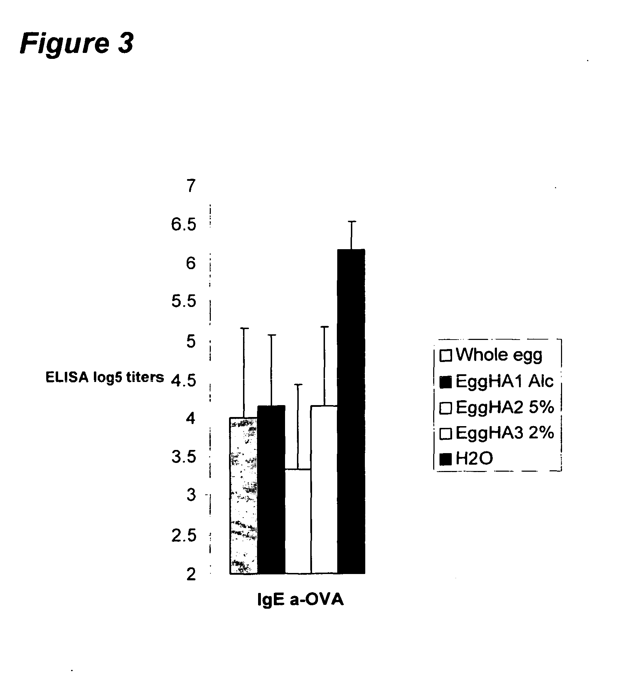

The results are shown in Figures 3, 4 and 5. From Figure 3, it may be seen that the egg protein hydrolysates from Examples 1 to 3 are able to suppress a specific IgE anti-egg protein response when fed to animals during 19 days *ad libitum* as compared to the non-tolerised control, Group F), which induced high levels of IgE anti-egg antibodies. More precisely, IgE anti OVA levels (expressed as log antibody titers) were as follows: Group A, 4+/- 1.1; Group B, 4.1 +/- 0.9; Group C, 3.3 +/- 1.1; Group D, 4.1 +/- 2.0; Group F, 6.1 +/- 0.3. When comparing groups, all of Groups A to D are significantly different (p<0.05) from group F (control).

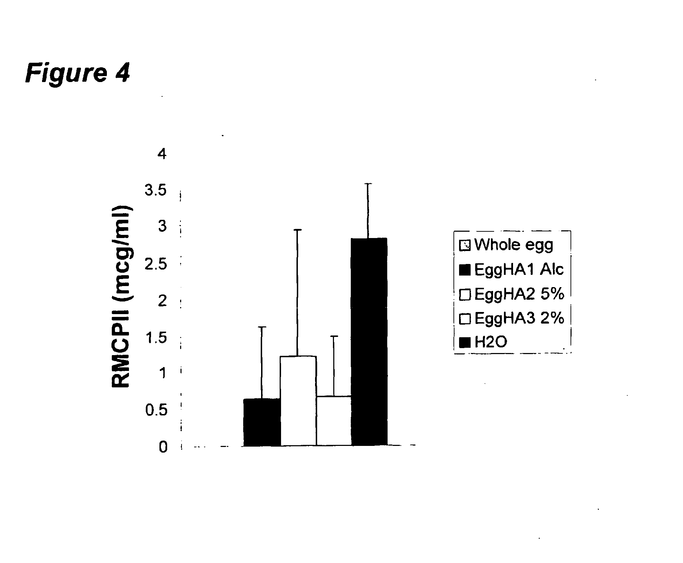

Figure 4 shows also that intestinal mast cell triggering is down-regulated in Groups A,B,C and D but not in Group F (control). Values, expressed in µg RMCPII /ml, are the following: Group A, 0 +/- 0.0; Group B, 0.6 +/- 1.0; Group C, 1.2 +/- 1.7; Group D, 0.6 +/- 0.8; Group F, 2.8 +/- 0.7. When comparing groups, all of Groups A to D are significantly different (p<0.05) from group F (control).

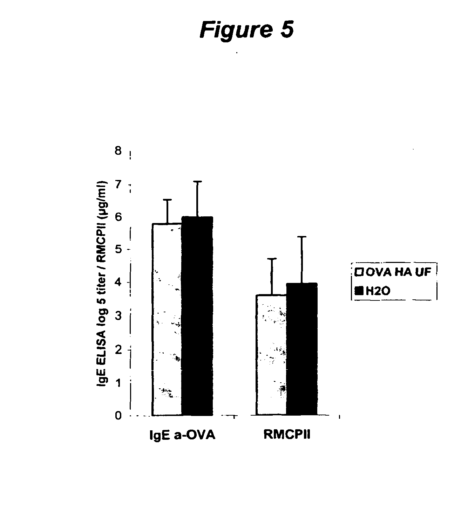

Figure 5 shows that the extensively hydrolysed egg proteins fed to Group E did not induce oral tolerance to OVA as measured by suppression of a specific IgE anti-egg protein response or down-regulation of intestinal mast cell triggering: IgE anti-OVA and RMCPII levels are not different from the values obtained for the control group.

## Claims

1. The use of enzymatically hydrolysed egg proteins with a degree of hydrolysis between 15 and 28% in the manufacture of a composition for induction of oral tolerance to egg proteins in a mammal.

2. The use of Claim 1, wherein the mammal is a human.

3. The use of Claim 2, wherein the composition is a weaning food for a baby.

4. The use of Claim 1, wherein the mammal is a companion animal such as a cat or a dog.

5. The use of any preceding claim, wherein the degree of hydrolysis is between 23 and 25%.

## Patentansprüche

1. Verwendung enzymatisch hydrolysierter Eiproteine mit einem Hydrolysegrad zwischen 15 und 28% in der Herstellung einer Zusammensetzung für die Induktion der oralen Verträglichkeit von Eiproteinen bei Säugern.

2. Verwendung nach Anspruch 1, wobei es sich bei den Säugern um Menschen handelt.

3. Verwendung nach Anspruch 2, wobei es sich bei der Zusammensetzung um Abstillnahrung für ein Baby handelt.

4. Verwendung nach Anspruch 1, wobei es sich bei dem Säuger um ein Haustier wie beispielsweise eine Katze oder einen Hund handelt.

5. Verwendung nach einem der vorhergehenden Ansprüche, wobei der Grad der Hydrolyse zwischen 23 und 25% liegt.

## Revendications

1. Utilisation des protéines d'oeuf hydrolysées enzymatiquement avec un degré d'hydrolyse entre 15 et 28 % dans la fabrication d'une composition pour l'induction de la tolérance orale aux protéines d'oeuf dans un mammifère.

2. Utilisation selon la revendication 1, le mammifère étant un homme.

3. Utilisation selon la revendication 2, la composition étant un aliment de sevrage pour un bébé.

4. Utilisation selon la revendication 1, le mammifère étant un animal de compagnie tel qu'un chat ou un chien.

5. Utilisation selon l'une quelconque des revendications précédentes, le degré d'hydrolyse étant entre 23 et 25 %.
